# EUROPÄISCHE PATENTSCHRIFT

(11) **EP 1 124 812 B1**
(45) Veröffentlichungstag und Bekanntmachung des Hinweises auf die Patenterteilung: **30.06.2004**
(21) Anmeldenummer: 99970989.2
(22) Anmeldetag: 26.10.1999
(51) Int. Cl.: C07D 301/16

(54) **VERFAHREN ZUR EPOXIDIERUNG VON OLEFINEN**
METHOD OF EPOXIDIZING OLEFINS
PROCEDE POUR L'EPOXYDATION D'OLEFINES

(30) Priorität: 27.10.1998 DE 19849527
(43) Veröffentlichungstag der Anmeldung: 22.08.2001
(73) Patentinhaber: BASF AKTIENGESELLSCHAFT, 67056 Ludwigshafen (DE)
(72) Erfinder: OTT, Christian, D-67346 Speyer (DE)
(74) Vertreter: Isenbruck, Günter
(86) Internationale Anmeldenummer: PCT/EP1999/008079
(87) Internationale Veröffentlichungsnummer: WO 2000/024726

(56) Entgegenhaltungen:
- EP-A- 0 032 990
- DE-A- 1 568 016
- US-A- 4 584 390
- US-A- 4 647 678

## Beschreibung

Die Erfindung betrifft ein Verfahren zur Epoxidierung von Olefinen mit in situ gebildeten Percarbonsäuren in einem zweiphasigen Reaktionssystem.

Epoxide olefinisch ungesättigter Verbindungen stellen eine bedeutende Zwischenproduktklasse dar. Die Epoxidierung der Olefine erfolgt in einem seit langem bekannten Verfahren durch Umsetzung der Olefine mit Percarbonsäuren, die in situ aus Wasserstoffperoxid und Carbonsäuren erzeugt werden können. Der an der Stelle der olefinischen Doppelbindung gebildete Oxiranring kann unter Ringöffnung mit einer Vielzahl von aktiven Wasserstoff enthaltenden Verbindungen reagieren und eröffnet eine umfangreiche Folgechemie.

Die Reaktivität der Epoxide führt in den Verfahren nach dem Stand der Technik zur Bildung von Nebenprodukten. So reagieren Epoxide in Gegenwart von Wasser und Carbonsäuren zu Glykolen bzw. deren Mono- und Diester, wobei die Öffnung des Oxiranringes säurekatalysiert verläuft. Problematisch ist dabei, daß auch die Bildung der Percarbonsäure säurekatalysiert ist, weshalb oft in Gegenwart von Mineralsäuren als Katalysatoren gearbeitet wird.

In DE-B 1 230 005 ist ein Verfahren zur Epoxidierung von linearen alpha-Olefinen offenbart, in dem als Epoxidierungsreagens wasser- und mineralsäurefreie Peressigsäure eingesetzt wird und in Gegenwart eines inerten Lösungsmittels wie Aceton, Methylacetat oder Ethylacetat gearbeitet wird. Nachteilig sind die langen Reaktionszeiten von mehreren Stunden, der unvollständige Olefinumsatz und der hohe Anteil an Nebenprodukten, insbesondere an Glykolmonoester. Auch ist das verwendete Epoxidierungsreagens teuer.

In DE-C 195 19 887 wird ein Verfahren zur Epoxidierung von olefinisch ungesättigten Verbindungen mit in situ hergestellter Percarbonsäure beschrieben, wobei mit Wasser als alleinigem Lösungsmittel und in Gegenwart von Inhibitoren gearbeitet wird. Über die genaue Zusammensetzung der Produktmischung werden keine Angaben gemacht.

Aus DE-A 15 68 016 ist ein Verfahren bekannt, bei dem alpha-Olefine in einem mit Wasser nicht mischbaren Lösungsmittel mit einer in der wäßrigen Phase in situ gebildeten Percarbonsäure epoxidiert werden, wobei das Reaktionsgemisch in der Weise gerührt wird, daß eine einzige Phasengrenzfläche aufrecht erhalten wird. Diese Verfahrensführung erfordert sehr lange Reaktionszeiten.

In EP-A 0 032 989 ist ein Verfahren zur Epoxidierung von alpha-Olefinen mit in situ gebildeter Perameisensäure beschrieben, bei dem in Abwesenheit eines Lösungsmittels und einer Katalysatorsäure gearbeitet wird. Die Epoxidierung nach diesem Verfahren erfordert eine lange Gesamtreaktionszeit. Auch ist trotz der langen Reaktionszeit der Olefinumsatz unbefriedigend.

US 4,584,390 offenbart ein mehrstufiges Verfahren zur kontinuierlichen Epoxidation von Olefinen, bei dem Perameisensäure in situ aus Wasserstoffperoxid und Ameisensäure erzeugt wird. Das zu epoxidierende Olefin und das Wasserstoffperoxid/Ameisensäure-Gemisch werden dabei im Kreuz-Gegenstrom durch eine dreistufige Reaktionskaskade geführt. Das Olefin liegt dabei nicht in einem organischen Lösungsmittel gelöst vor. Das wässrige Wasserstoffperoxid/Ameisensäure-Gemisch wird nicht nach jeder Reaktionsstufe erneuert.

Aufgabe der Erfindung ist es, ein Verfahren zur Epoxidierung von Olefinen bereitzustellen, bei dem nach kurzen Reaktionszeiten ein quantitativer Umsatz erzielt und Produkte hoher Reinheit erhalten werden.

Gelöst wird die Aufgabe durch ein Verfahren zur Epoxidierung von Olefinen mit Percarbonsäure in einer Reaktionsmischung aus einer wäßrigen Phase und einer organischen Phase, wobei die Percarbonsäure in der wäßrigen Phase in situ aus Wasserstoffperoxid und einer Carbonsäure oder einem Carbonsäureanhydrid gebildet wird und die Olefine in der organischen Phase in einem organischen Lösungsmittel gelöst vorliegen. Das erfindungsgemäße Verfahren ist dadurch gekennzeichnet, daß die Epoxidierung in mehreren Schritten durchgeführt wird, wobei jeder Schritt mit jeweils einer frischen wäßrigen Phase durchgeführt und nach jedem Schritt die wäßrige Phase abgetrennt wird. wobei jeder Schritt mit jeweils einer frischen wäßrigen Phase durchgeführt und nach jedem Schritt die wäßrige Phase abgetrennt wird.

Olefine im Sinne der vorliegenden Erfindung sind Verbindungen, die eine oder mehrere olefinische Doppelbindungen enthalten. Verbindungen mit olefinischen Doppelbindungen sind beispielsweise lineare oder verzweigte Mono- und Diolefine mit 6 bis 30 C-Atomen, ungesättigte, gegebenenfalls hydroxysubstituierte Fettsäuren mit 6 bis 24 C-Atomen und 1 bis 5 Doppelbindungen, deren Ester bzw. Triglyceride sowie ungesättigte Alkohole mit 6 bis 24 C-Atomen und 1 bis 3 Doppelbindungen. Auch Polyalkene und Terpene sind Olefine im Sinne der vorliegenden Erfindung. Die Olefine können neben der oder den Doppelbindungen weitere funktionelle Gruppen aufweisen, die unter den Reaktionsbedingungen nicht oder nur langsam mit dem Lösungsmittel oder dem Epoxidierungsreagens reagieren. Beispielsweise können die Olefine Heteroatome, wie einen Ether-Sauerstoff, enthalten, oder mit Hydroxygruppen, Carbonsäuregruppen, Carbonsäureamid-, Carbonsäureimid-, Carbonsäureester-, Lactam- oder Lactongruppen, aromatischen Resten oder Halogenatomen substituiert sein. Das eingesetzte Olefin kann bereits Epoxidgruppen, Ketogruppen oder cyclische Carbonatgruppen aufweisen.

Geeignete Olefine sind beispielsweise Mono- oder Diolefine mit 6 bis 30, bevorzugt 10 bis 24 C-Atome, besonders bevorzugt 12 bis 18 C-Atome, die verzweigt oder unverzweigt sein können. Verzweigte Olefine können auch an den Doppelbindungen verzweigt sein. Es können sowohl cis- als auch trans-Isomere eingesetzt werden. Beispiele sind 1-Hexen, 2-Hexen, 3-Hexen, 1,3-Hexadien, 1,4-Hexadien, 3-Methyl-1,3-pentadien, 2-Methyl-1-penten, 3,3-Dimethyl-1-buten, 1-Hepten, 3-Hepten, 1-Octen, 4-Octen, 1-Nonen, 1-Decen, 1-Undecen, 1-Dodecen, 1-Tetradecen, 1-Hexadecen, 1-Octadecen, 1-Eicosen. Bevorzugt sind lineare Olefine, beispielsweise die oben genannten linearen Olefine, von denen solche mit endständiger Doppelbindung (lineare alpha-Olefine) besonders bevorzugt sind. Von den linearen alpha-Olefinen sind solche mit geradzahliger Anzahl von C-Atomen wegen ihrer leichten Verfügbarkeit aus petrochemischen Prozessen besonders bevorzugt.

Geeignete Olefine sind ferner cyclische Olefine, wie Cyclohexen, Cycloocten, Cyclooctadien, Cyclodecen und Cyclododecen sowie deren substituierte Vertreter, beispielsweise substituierte Cyclohexene wie 1,3-Dimethylcyclohexen, 1,4-Dimethylcyclohexen oder 1-Ethylcyclohexen.

Geeignete Olefine sind ferner Terpene, Terpenalkohole sowie weitere Naturstoffe mit einer oder mehreren Doppelbindungen, wie Steroide. Diese können neben den olefinischen Doppelbindungen weitere funktionelle Gruppen wie Hydroxy- oder Ketogruppen aufweisen. Beispiele sind 2-Caren, delta-3-Caren, alpha-Pinen, beta-Pinen, Verbenol, Myrtenol, cis-Jasmon, Dihydrocarveol, alpha-Terpinen, gamma-Terpinen, alpha-Ionon, beta-Ionon, Limonen, Carvon, Citronellinsäure, trans-Vacceninsäure, Geraniol, Farnesol, Phytol, Citronellol, Ergosterol, Myrcen, Squalen und Camphen.

Geeignete Olefine sind auch ungesättigte Fettsäuren mit 6 bis 24 C-Atomen mit 1 bis 5 Doppelbindugen, die gegebenenfalls mit Hydroxygruppen substituiert sein können, beispielsweise Ölsäure, Elaidinsäure, Linolsäure, Linolensäure, Arachidonsäure, Linolenelaidinsäure, Linoelaidinsäure, Myristoleinsäure, Palmitoleinsäure, Undecyleinsäure, oder Ricinolsäure. Geeignete Olefine sind auch die Ester der ungesättigten Fettsäuren, insbesondere deren Triglyceride, wie sie in tierischen und pflanzlichen Fetten und Ölen vorkommen, beispielsweise in Sojaöl, Sonnenblumenöl, Leinöl, Rapsöl, Rüböl, Erdnußöl, Palmöl, Kokosöl, Ricinusöl, Rindertalg, Schweineschmalz und Fischöl. Geeignete Olefine sind ferner ungesättigte Fettalkohole mit 1 bis 3 Doppelbindungen, wie Oleylalkohol, Elaidylalkohol, Linoleylalkohol, Linolenylalkohol und Arachidonalkohol.

Bevorzugte Olefine sind auch Polyalkylene wie Polyisobuten.

Die Olefine liegen in einem organischen Lösungsmittel gelöst vor. Geeignete organische Lösungsmittel bilden mit dem Epoxidierungsreagenz aus einer Carbonsäure und wäßriger Wasserstoffperoxidlösung ein zweiphasiges Reaktionssystem aus. Geeignete organische Lösungsmittel sind daher mit Wasser nicht mischbar oder mit Wasser nur begrenzt mischbare organische Lösungsmittel. Die organischen Lösungsmittel werden in einer Menge eingesetzt, bei der es zur Ausbildung einer von der wäßrigen Phase getrennten organischen Phase kommt. Daher sind mit Wasser vollständig mischbare Lösungsmittel nicht geeignet.

Bevorzugte organische Lösungsmittel sind weiterhin gegenüber wäßriger Wasserstoffperoxidlösung, Carbonsäuren und Percarbonsäuren und insbesondere gegenüber den gebildeten Epoxiden inert. Bevorzugte Lösungsmittel weisen eine zu Wasser, Wasserstoffperoxidlösung bzw. der wäßrigen Carbonsäurelösung deutlich verschiedene Dichte auf.

Bevorzugte organische Lösungsmittel sind mit Wasser zumindest begrenzt mischbar. Vorzugsweise weist das organische Lösungsmittel bei 25°C ein Wasseraufnahmevermögen von mindestens 0,1 und höchstens 10 mol-%, besonders bevorzugt mindestens 0,1 und höchstens 5 mol-%, insbesondere mindestens 0,2 und höchstens 2 mol-%, speziell mindestens 0,2 und höchstens 1,0 mol-%.

Geeignete Lösungsmittel sind beispielsweise aliphatische und aromatische Kohlenwasserstoffe und Halogenkohlenwasserstoffe wie Pentane, Hexane, Heptane, Octane, Cyclopentan, Cyclohexan, Methylcyclopentan, Methylcyclohexan, Dichlormethan, Trichlormethan, Tetrachlormethan, Trichlorethan, Benzol, Toluol, Xylole, Ethylbenzol, Chlorbenzol und Dichlorbenzole, ferner aliphatische und alicyclische Ether wie Diethylether, Dipropylether, Diisopropylether, Tetrahydrofuran und Dioxan. Besonders bevorzugt sind wegen ihres ausreichend hohen, aber begrenzten Wasseraufnahmevermögens und ihrer geringen Reaktivität aromatische Kohlenwasserstoffe wie Benzol, Chlorbenzol, Toluol, ortho-, meta- und para-Xylol, Ethylbenzol und halogenierte Kohlenwasserstoffe wie Dichlormethan, Chloroform und Chlorbenzol. Das Gewichtsverhältnis Olefin : Lösungsmittel liegt im allgemeinen im Bereich von 1 : 100 bis 10 : 1, bevorzugt von 1:10 bis 2:1, besonders bevorzugt von 1:5 bis 1:1.

Die Percarbonsäure wird in der wäßrigen Phase in situ aus einer Carbonsäure oder einem Carbonsäureanhydrid und wäßriger Wasserstoffperoxidlösung gebildet. Geeignete Carbonsäuren sind Carbonsäuren, die bei der Reaktionstemperatur in der wäßrigen Phase zumindest teilweise löslich sind, wie mit Wasser zumindest teilweise mischbare aliphatische, araliphatische und aromatische Carbonsäuren. Beispiele sind Ameisensäure, Essigsäure, Chloressigsäure, Dichloressigsäure, Trichloressigsäure, Phenylessigsäure, Trifluoressigsäure, Propionsäure, Benzoesäure, meta-Chlorbenzoesäure und Phthalsäure. Es können auch Carbonsäureanhydride, beispielsweise Maleinsäureanhydrid, eingesetzt werden. Bevorzugte Carbonsäuren weisen in Wasser eine hohe Löslichkeit und in den eingesetzten organischen Lösungsmitteln eine geringe Löslichkeit auf. Besonders bevorzugte Carbonsäuren sind mit Wasser unbegrenzt mischbar. Bevorzugt sind beispielsweise aliphatische Carbonsäuren mit 1 bis 3 C-Atomen wie Ameisensäure, Essigsäure, Propionsäure. Bevorzugt ist ferner Maleinsäureanhydrid. Besonders bevorzugt sind Essigsäure und Ameisensäure, insbesondere bevorzugt ist Ameisensäure.

Als Wasserstoffperoxidlösung wird im allgemeinen eine 3 bis 95 gew.-%ige, bevorzugt eine 10 bis 90 gew.-%ige, besonders bevorzugt eine 30 bis 85 gew.-%ige, insbesondere eine 50 bis 80 gew.-%ige wäßrige Wasserstoffperoxidlösung eingesetzt.

Die Epoxidierung wird in mehreren Schritten durchgeführt, wobei in jedem Schritt mit einer frischen wäßrigen Phase gearbeitet wird. Es kann, bezogen auf jeweils einen Schritt, die gesamte Carbonsäuremenge auf einmal oder nur ein Teil der Carbonsäure vorgelegt und gegebenenfalls der Rest der Carbonsäure und die Wasserstoffperoxidlösung portionsweise oder kontinuierlich zugegeben werden. Vorzugsweise wird nach der ersten Variante verfahren, wobei es besonders bevorzugt ist, die gesamte Carbonsäuremenge vorzulegen und die Wasserstoffperoxidlösung kontinuierlich zulaufen zu lassen. Die wäßrige Phase wird in jedem Schritt, vorzugsweise durch Rühren, in der organischen Phase unter Tröpfchenbildung dispergiert.

Das Molverhältnis der pro Schritt eingesetzten Menge an Wasserstoffperoxid und Carbonsäure ist (bezogen auf Monocarbonsäuren) liegt allgemein im Bereich von 100 : 1 bis 1 : 10, bevorzugt von 10 : 1 bis 1 : 2, besonders bevorzugt von 5 : 1 bis 1 : 2, insbesondere bevorzugt von 2 : 1 bis 1 : 2. Dieses Verhältnis kann von Schritt zu Schritt unterschiedlich sein.

Das Molverhältnis der in allen Schritten insgesamt eingesetzten Menge an Wasserstoffperoxid zu der eingesetzten Olefinmenge ist im allgemeinen mindestens stöchiometrisch, das heißt (bezogen auf Monoolefine) mindestens 1 : 1, bevorzugt 5 : 1 bis 1 : 1, besonders bevorzugt 2 : 1 bis 1 : 1.

Vorzugsweise wird die Epoxidierung in zwei bis vier Schritten, besonders bevorzugt in drei oder vier Schritten durchgeführt. Die pro Schritt eingesetzte Wasserstoffperoxidmenge kann gleichbleiben oder variieren. Bevorzugt wird die pro Schritt eingesetzte Wasserstoffperoxidmenge mit zunehmendem Olefinumsatz abnehmen. Die pro Schritt eingesetzte Carbonsäuremenge kann ebenfalls gleichbleiben oder variieren. Bevorzugt wird die pro Schritt eingesetzte Carbonsäuremenge mit zunehmendem Olefinumsatz von Schritt zu Schritt abnehmen, insbesondere dann, wenn auch die pro Schritt eingesetzte Wasserstoffperoxidmenge von Schritt zu Schritt abnimmt.

In einer bevorzugten Ausführungsform des erfindungsgemäßen Verfahrens wird im ersten Schritt eine bezüglich Olefin stöchiometrische Menge Wasserstoffperoxid, d. h. pro Mol olefinische Doppelbindungen ein Mol Wasserstoffperoxid, und in jedem weiteren Schritt eine geringere Menge, insgesamt jedoch nicht mehr als zwei Mol, besonders bevorzugt nicht mehr als 1,5 Mol Wasserstoffperoxid, eingesetzt.

In einer weiteren bevorzugten Ausführungsform wird im ersten Schritt eine bezüglich Olefin unterstöchiometrische Menge Wasserstoffperoxid, bevorzugt pro Mol olefinischer Doppelbindung 0,5 Mol Wasserstoffperoxid, und in jedem weiteren Schritt eine geringere Menge, insgesamt jedoch nicht mehr als 1,5 Mol, besonders bevorzugt nicht mehr als 1,2 Mol Wasserstoffperoxid, eingesetzt.

In einer weiteren bevorzugten Ausführungsform wird die Epoxidierung derart durchgeführt, daß nach dem ersten Schritt im allgemeinen 30 bis 90 %, bevorzugt 30 bis 70 % des Olefins umgesetzt sind. Wird das erfindungsgemäße Verfahren in mindestens drei Schritten durchgeführt, so ist der Olefinumsatz nach dem zweiten Schritt im allgemeinen 50 bis 95 %, bevorzugt 60 bis 90 %. Der Fortgang der Reaktion kann gaschromatographisch oder titrimetrisch, beispielsweise durch Bestimmung der Iodzahl oder des Epoxidgehaltes, verfolgt werden und ein Epoxidierungsschritt bei Erreichen eines bestimmten. Umsatzes durch Phasentrennung abgebrochen werden.

Nach jedem Schritt wird die wäßrige Phase abgetrennt und durch eine frische wäßrige Phase ersetzt. Dabei ist nicht unbedingt erforderlich, daß die Percarbonsäure und/oder Wasserstoffperoxid vollständig abreagiert haben. Dies ist aber im Sinne einer effizienten Ausnutzung der Persäure gewünscht.

Die Epoxidierungsreaktion wird vorzugsweise bei Atmosphärendruck durchgeführt. Die Umsetzungstemperatur richtet sich nach der Kettenlänge des Olefins und beträgt im allgemeinen von 60 bis 100 °C. Die Reaktion kann in üblichen Reaktionsapparaten, wie einem Rührkessel oder einer Rührkesselkaskade, mit üblichen Einrichtungen zur Phasentrennung durchgeführt werden. Die Reaktion wird bevorzugt unter Rückfluß durchgeführt, wobei der eingesetzte Kondensator für eine ausreichende Wärmeabfuhr sorgt.

Das erfindungsgemäße Verfahren weist eine Reihe von Vorteilen auf. So ist die Gesamtreaktionszeit der Epoxidierungsreaktion kurz. Sie beträgt, bei einem Umsatz von mindestens 95 Gew.-% Olefin, bevorzugt von mindestens 98 Gew.-%, im allgemeinen < 10 h, bevorzugt < 7 h, besonders bevorzugt < 5 h. Dabei beträgt die Reaktionszeit pro Schritt bevorzugt < 1 h.

Bei dem erfindungsgemäßen Verfahren wird ein Epoxid hoher Reinheit gebildet. Im allgemeinen ist der Epoxidgehalt des Rohproduktes, bezogen auf eingesetztes Olefin, > 90 mol-%, bevorzugt > 95 mol-%. Der Gehalt an Glykol ist im allgemeinen < 5 Gew.-%, bevorzugt < 2 Gew.-%, der Gehalt an weiteren Nebenbestandteilen, wie den Glykolmonoestern und Diestern der eingesetzten Carbonsäuren, im allgemeinen insgesamt < 5 mol-%, bevorzugt < 2 mol-%.

Durch den Austausch der wäßrigen Phase werden eine ausreichend hohe Carbonsäurekonzentration und Wasserstoffperoxidkonzentration in der wäßrigen Phase aufrecht erhalten, so daß auf den Einsatz von Mineralsäuren als Katalysatoren für die Peroxidbildung verzichtet werden.

Durch das Arbeiten in einem zweiphasigen Reaktionssystem werden einerseits Folgereaktionen der in der organischen Phase vorliegenden Epoxide mit Wasser oder der in der wässrigen Phase vorliegenden Carbonsäure, beispielsweise die säurekatalysierte Ringöffnung des Oxiranrings unter Bildung von Glykolen, Monoestern und Diestern, weitgehend unterbunden. Durch die Reduzierung der Carbonsäuremenge in der wäßrigen Phase mit fortschreitendem Olefinumsatz kann die Gefahr der Bildung von Folgeprodukten zusätzlich verringert werden.

Durch die Wahl eines geeigneten Lösungsmittels mit ausreichend hoher Polarität (Wasseraufnahmevermögen) wird andererseits ein effektiver Transport der Percarbonsäure in die organische Phase und damit eine ausreichend hohe Reaktionsgeschwindigkeit gewährleistet.

Das erhaltene Produkt kann, gegebenenfalls nach Abdestillieren des Lösungsmittels, ohne weitere Aufreinigung weiterverwendet werden. Bei Verwendung von beispielsweise Toluol als organischem Lösungsmittel und Ameisensäure als Carbonsäure werden gegebenenfalls vorhandene Spuren von Ameisensäure und Wasser als Azeotrop herausgeschleppt, so daß ein weitgehend trockenes und säurefreies Produkt resultiert. Entsprechendes gilt für die Systeme Toluol/Essigsäure/Wasser, Xylol/Essigsäure/Wasser, Xylol/Ameisensäure/Wasser und Dichlormethan/Ameisensäure/Wasser. Bei sehr hohen Anforderungen an die Produktreinheit kann eine Feindestillation im Vakuum durchgeführt werden.
Die Erfindung wird durch die nachfolgenden Beispiele näher erläutert.

### Beispiele

### Beispiel 1

In einem 2 l-Kolben werden 336 g 1-Dodecen (2,0 mol), 800 g Toluol, 46 g konzentrierter Ameisensäure (99 Gew.-%, 1,0 mol) vorgelegt und auf 90°C erwärmt. Zu der intensiv gerührten Mischung werden in einem ersten Schritt über einen Zeitraum von 20 min 136 g Wasserstoffperoxidlösung (50 Gew.-%, 2,0 mol) zugetropft, anschließend wird noch 20 min weitergerührt. Die wäßrige Phase wird abgetrennt. Im zweiten bis vierten Schritt werden die angegebenen Mengen Ameisensäure auf einmal zugegeben und die angegebenen Mengen Wasserstoffperoxid (Konzentrationen und Temperatur wie oben) über einen Zeitraum von jeweils 5 min zugetropft, jeweils 20 min weitergerührt und die wäßrige Phase anschließend abgetrennt.
2. Schritt: 0,5 mol Ameisensäure und 0,5 mol Wasserstoffperoxid;
3. und 4. Schritt: jeweils 0,3 mol Ameisensäure und 0,3 mol Wasserstoffperoxid;
Es wird ein Rohprodukt folgender Zusammensetzung erhalten (in GC-Flächen-%): 1,2-Epoxydodecan 93,2 %, 1-Dodecen 1,1 %, 1,2-Dodecandiol 2,1 %, 1,2-Dodecandiolmonoformylester und 1,2-Dodecandioldiformylester 1,6 %.

### Beispiel 2

In einem 1 l-Kolben werden 168 g 1-Dodecen (1,0 mol), 400 g Dichlormethan, 96 g konzentrierter Ameisensäure (99 Gew.-%, 1,5 mol) vorgelegt und auf 90°C erwärmt. Zu der intensiv gerührten Mischung werden in einem ersten Schritt über einen Zeitraum von 5 min 102 g Wasserstoffperoxidlösung (50 Gew.-%, 1,5 mol) zugetropft, anschließend wird noch 45 min weitergerührt. Die wäßrige Phase wird abgetrennt. Im zweiten und dritten Schritt werden die angegebenen Mengen Ameisensäure auf einmal zugegeben und die angegebenen Mengen Wasserstoffperoxid (Konzentrationen und Temperatur wie oben) über einen Zeitraum von jeweils 5 min zugetropft, jeweils 45 min weitergerührt und die wäßrige Phase anschließend abgetrennt.
2. Schritt: 0,5 mol Ameisensäure und 0,5 mol Wasserstoffperoxid;
3. Schritt: 0,3 mol Ameisensäure und 0,3 mol Wasserstoffperoxid;
Es wird ein Rohprodukt mit 94,5 Gew.-% 1,2-Epoxydodecan erhalten.

### Beispiel 3

In einem 5 l-Kolben werden 2295,3 g Polyisobuten 1000 (3,0 mol), 1200 g Toluol, 92 g konzentrierter Ameisensäure (99 Gew.-%, 2,0 mol) vorgelegt und auf 90°C erwärmt. Zu der intensiv gerührten Mischung werden in einem ersten Schritt über einen Zeitraum von 40 min 136 g Wasserstoffperoxidlösung (50 Gew.-%, 2,0 mol) zugetropft, anschließend wird noch 45 min weitergerührt. Die wäßrige Phase wird abgetrennt. Im zweiten Schritt werden 0,5 mol Ameisensäure auf einmal zugegeben, 0,5 mol Wasserstoffperoxid (Konzentrationen und Temperatur wie oben) über einen Zeitraum von 20 min zugetropft, 60 min weitergerührt und die wäßrige Phase anschließend abgetrennt. Das Epoxid wird in quantitativer Ausbeute erhalten.

### Beispiel 4:

In einem 5 l-Reaktor werden 2295,3 g Polyisobuten 1000 (3,0 mol), 1200 g Toluol, 92 g konzentrierter Ameisensäure (99 Gew.-%, 2,0 mol) vorgelegt und auf 90°C erwärmt. Zu der intensiv gerührten Mischung werden in einem ersten Schritt über einen Zeitraum von 40 min 136 g Wasserstoffperoxidlösung (50 Gew.-%, 2,0 mol) zugetropft, anschließend wird noch 60 min weitergerührt. Die wäßrige Phase wird abgetrennt. Im zweiten Schritt werden 46 g (1,0 mol) Ameisensäure auf einmal zugegeben, 68 g (1,0 mol) Wasserstoffperoxid (Konzentrationen und Temperatur wie oben) über einen Zeitraum von 20 min zugetropft, 60 min weitergerührt und die wäßrige Phase anschließend abgetrennt und die organische Phase im Vakuum eingeengt. Das Epoxid wird in quantitativer Ausbeute erhalten.

### Beispiel 5:

In einem 10 l-Reaktor werden 2295,3 g Polyisobuten 1000 (3,0 mol), 983,7 g Mihagol®M (ein Gemisch aus C₁₀-C₁₄ n-Paraffinen), 92 g konzentrierter Ameisensäure (99 Gew.-%, 2,0 mol) vorgelegt und auf 90°C erwärmt. Zu der intensiv gerührten Mischung werden in einem ersten Schritt über einen Zeitraum von 20 min 136 g Wasserstoffperoxidlösung (50 Gew.-%, 2,0 mol) zugetropft, anschließend wird noch 60 min weitergerührt. Die wäßrige Phase wird abgetrennt. Im zweiten Schritt werden 46 g (1,0 mol) Ameisensäure auf einmal zugegeben, 1,0 mol Wasserstoffperoxid (Konzentrationen und Temperatur wie oben) über einen Zeitraum von 10 min zugetropft, 60 min weitergerührt und die wäßrige Phase anschließend abgetrennt. Anschließend werden zu der organischen Phase 300 ml Toluol zugegeben und es wird zur Entfernung von Wasser und Ameisensäure destilliert. Schließlich wird die organische Phase im Vakuum eingeengt. Das Epoxid wird in quantitativer Ausbeute erhalten.

### Vergleichsbeispiel VI:

168 g 1-Dodecen (1,0 mol) werden vorgelegt und mit 92 g konzentrierter Ameisensäure (99 Gew.-%, 2,0 mol) gemischt. Zu der intensiv gerührten Mischung werden bei 90 °C 81,6 g Wasserstoffperoxid (50 Gew.-%, 1,2 mol) über einen Zeitraum von 15 min zugetropft, anschließend wird bei 90 °C noch 2 h lang intensiv weitergerührt. Der Reaktionsverlauf wird mittels Titration der Persäure/Peroxidmenge verfolgt. Nach beendeter Reaktion werden die Phasen bei 80 °C getrennt und die organische Phase gaschromatographisch analysiert.

Es werden 209 g eines niedrig schmelzenden farblosen Feststoffes folgender Zusammensetzung (in GC-Flächen-%) erhalten: 1,2-Dodecandiol 73,4 %, 1,2-Dodecandiol-1-monoformylester 9,8 %, 1,2-Dodecandiol-2-monoformylester 15,9 %, 1,2-Dodecandioldiformylester 0,9 %.

## Patentansprüche

1. Verfahren zur Epoxidierung von Olefinen mit Percarbonsäure in einer Reaktionsmischung aus einer wäßrigen Phase und einer organischen Phase, wobei die Percarbonsäure in der wäßrigen Phase in situ aus Wasserstoffperoxid und einer Carbonsäure oder einem Carbonsäureanhydrid gebildet wird und die Olefine in der organischen Phase in einem organischen Lösungsmittel gelöst vorliegen, **dadurch gekennzeichnet, daß** die Epoxidierung in mehreren Schritten durchgeführt wird, wobei jeder Schritt mit jeweils einer frischen wäßrigen Phase durchgeführt und nach jedem Schritt die wäßrige Phase abgetrennt wird.

2. Verfahren nach Anspruch 1, **dadurch gekennzeichnet, daß** das Wasseraufnahmevermögen des organischen Lösungsmittels bei 25 °C mindestens 0,1 und höchstens 10 mol-% beträgt.

3. Verfahren nach Anspruch 1 oder 2, **dadurch gekennzeichnet, daß** das organische Lösungsmittel ausgewählt ist aus Benzol, Toluol, ortho-, meta- und para-Xylol, Ethylbenzol und halogenierten Kohlenwasserstoffen.

4. Verfahren nach einem der Ansprüche 1 bis 3, **dadurch gekennzeichnet, daß** die Carbonsäure bzw. das Carbonsäureanhydrid ausgewählt sind aus Ameisensäure, Essigsäure, Propionsäure und Maleinsäureanhydrid.

5. Verfahren nach einem der Ansprüche 1 bis 4, **dadurch gekennzeichnet, daß** die Carbonsäure Ameisensäure ist.

6. Verfahren nach einem der Ansprüche 1 bis 5, **dadurch gekennzeichnet, daß** lineare alpha-Olefine mit 6 bis 30 C-Atomen epoxidiert werden.

7. Verfahren nach einem der Ansprüche 1 bis 6, **dadurch gekennzeichnet, daß** die pro Schritt eingesetzte Wasserstoffperoxidmenge von Schritt zu Schritt abnimmt.

8. Verfahren nach einem der Ansprüche 1 bis 7, **dadurch gekennzeichnet, daß** die pro Schritt eingesetzte Säuremenge von Schritt zu Schritt abnimmt.

9. Verfahren nach einem der Ansprüche 1 bis 5, **dadurch gekennzeichnet, daß** die Epoxidierung in mindestens drei Schritten derart durchgeführt wird, daß nach dem ersten Schritt 30 bis 70 % und nach dem zweiten Schritt 50 bis 90 % des Olefins umgesetzt sind.

10. Verfahren nach einem der Ansprüche 1 bis 9, **dadurch gekennzeichnet**, die Epoxidierung bei Atmosphärendruck und einer Temperatur von 60 bis 100 °C durchgeführt wird.

11. Verfahren nach einem der Ansprüche 1 bis 10, **dadurch gekennzeichnet, daß** Polyisobuten expoxidiert wird.

## Claims

1. A process for the epoxidation of olefins with percarboxylic acid in a reaction mixture consisting of an aqueous phase and an organic phase, the percarboxylic acid being formed in situ in the aqueous phase from hydrogen peroxide and a carboxylic acid or a carboxylic anhydride, and the olefins being dissolved in an organic solvent in the organic phase, which process comprises carrying out the epoxidation in several steps, each step being carried out with a fresh aqueous phase and the aqueous phase being separated off after each step.

2. A process as claimed in claim 1, wherein the water absorption capacity of the organic solvent at 25°C is at least 0.1 and at most 10 mol%.

3. A process as claimed in claim 1 or 2, wherein the organic solvent is selected from benzene, toluene, ortho-, meta- and para-xylene, ethylbenzene and halogenohydrocarbons.

4. A process as claimed in any of claims 1 to 3, wherein the carboxylic acid or carboxylic anhydride is selected from formic acid, acetic acid, propionic acid and maleic anhydride.

5. A process as claimed in any of claims 1 to 4, wherein the carboxylic acid is formic acid.

6. A process as claimed in any of claims 1 to 5, wherein linear alpha-olefins having from 6 to 30 C atoms are epoxidized.

7. A process as claimed in any of claims 1 to 6, wherein the amount of hydrogen peroxide used per step decreases from step to step.

8. A process as claimed in any of claims 1 to 7, wherein the amount of acid used per step decreases from step to step.

9. A process as claimed in any of claims 1 to 5, wherein the epoxidation is carried out in at least three steps in such a way that the olefin conversion is 30 to 70% after the first step and 50 to 90% after the second step.

10. A process as claimed in any of claims 1 to 9, wherein the epoxidation is carried out under atmospheric pressure and at a temperature of 60 to 100°C.

11. A process as claimed in any of claims 1 to 10, wherein polyisobutene is epoxidized.

## Revendications

1. Procédé d'époxydation d'oléfines par de l'acide percarboxylique dans un mélange réactionnel à base d'une phase aqueuse et d'une phase organique, dans lequel l'acide percarboxylique est formé dans la phase aqueuse in situ à partir de peroxyde d'hydrogène et d'un acide carboxylique ou d'un anhydride d'acide carboxylique et les oléfines se présentent en solution dans un solvant organique dans la phase organique, **caractérisé en ce que** l'époxydation est effectuée en plusieurs étapes, chaque étape étant effectuée avec une phase aqueuse fraîche, la phase aqueuse étant isolée après chaque étape.

2. Procédé suivant la revendication 1, **caractérisé en ce que** la capacité d'absorption d'eau du solvant organique à 25°C est d'au minimum 0,1 et d'au maximum 10 moles %.

3. Procédé suivant l'une des revendications 1 et 2, **caractérisé en ce que** le solvant organique est choisi parmi du benzène, du toluène, de l'ortho-xylène, du méta-xylène, du para-xylène, de l'éthyl-benzène et des hydrocarbures halogénés.

4. Procédé suivant l'une des revendications 1 à 3, **caractérisé en ce que** l'acide carboxylique ou respectivement l'anhydride d'acide carboxylique est choisi parmi de l'acide formique, de l'acide acétique, de l'acide propionique et de l'anhydride maléique.

5. Procédé suivant l'une des revendications 1 à 4, **caractérisé en ce que** l'acide carboxylique est de l'acide formique.

6. Procédé suivant l'une des revendications 1 à 5, **caractérisé en ce que** des alpha-oléfines linéaires comportant 6 à 30 atomes de C sont époxydées.

7. Procédé suivant l'une des revendications 1 à 6, **caractérisé en ce que** la quantité de peroxyde d'hydrogène mise en oeuvre par étape diminue d'étape en étape.

8. Procédé suivant l'une des revendications 1 à 7, **caractérisé en ce que** la quantité d'acide mise en oeuvre par étape diminue d'étape en étape.

9. Procédé suivant l'une des revendications 1 à 5, **caractérisé en ce que** l'époxydation est effectuée en au moins trois étapes de façon que 30 à 70% de l'oléfine soient convertis après la première étape et 50 à 90% après la deuxième étape.

10. Procédé suivant l'une des revendications 1 à 9, **caractérisé en ce que** l'époxydation est effectuée à la pression atmosphérique et à une température de 60 à 100°C.

11. Procédé suivant l'une des revendications 1 à 10, **caractérisé en ce que** du polyisobutène est époxydé.
